# EUROPÄISCHE PATENTSCHRIFT

(11) **EP 0 888 313 B1**
(45) Veröffentlichungstag und Bekanntmachung des Hinweises auf die Patenterteilung: **17.04.2002**
(21) Anmeldenummer: 97915277.4
(22) Anmeldetag: 29.01.1997
(51) Int. Cl.: C07D 243/02, A61K 31/55

(54) **2,3-BENZODIAZEPINDERIVATE UND DEREN VERWENDUNG ALS AMPA-REZEPTOREN-HEMMER**
2,3-BENZODIAZEPINE DERIVATIVES AND THEIR USE AS AMPA-RECEPTOR INHIBITORS
DERIVES DE 2,3-BENZODIAZEPINE ET LEUR UTILISATION COMME INHIBITEURS DE RECEPTEURS D'AMPA

(30) Priorität: 01.02.1996 DE 19604920
(43) Veröffentlichungstag der Anmeldung: 07.01.1999
(73) Patentinhaber: SCHERING AKTIENGESELLSCHAFT, 13353 Berlin (DE)
(72) Erfinder: LING, István, H-1141 Budapest (HU); ABRAHAM, Gizella, H-1118 Budapest (HU); SOLYOM, Sándor, H-1144 Budapest (HU); HAMORI, Tamás, H-1031 Budapest (HU); TARNAWA, István, H-1147 Budapest (HU); BERZSENYI, Pál, H-1174 Budapest (HU); ANDRASI, Ferenc, H-1125 Budapest (HU); CSUZDI, Emese, H-1046 Budapest (HU); SZÖLLOSY, Márta, H-1105 Budapest (HU); SIMAY, Antal, H-1124 Budapest (HU); PALLAGI, István, H-1157 Budapest (HU); HORVATH, Katalin, H-1025 Budapest (HU)
(86) Internationale Anmeldenummer: DE9700225
(87) Internationale Veröffentlichungsnummer: WO9728135

(56) Entgegenhaltungen:
- EP-A- 0 512 419
- GB-A- 2 034 706
- GB-A- 2 162 184
- US-A- 4 423 044
- EUROPEAN JOURNAL OF PHARMACOLOGY, Bd. 294, 1995, AMSTERDAM NL, Seiten 411-22, XP002034232 G. DE SARRO ET AL.: "GYKI 52466 and related 2,3-benzodiazepines as anticonvulsant agents in DBA/2 mice"

## Beschreibung

Die Erfindung betrifft neue 8-Alkoxy-substituierte 2,3-Benzodiazepinderivate, deren Herstellung und Verwendung als Arzneimittel.

Es ist aus EP 0 512 419 A, Eur. J. Pharmacol., 294 (1995), 411 - 422,
GB 2 034 706 A, US 4 423 044 A und GB 2 162 184 A bekannt, daß ausgewählte 2,3-Benzodiazepinderivate als Arzneimittel zur Behandlung von Krankheiten des zentralen Nervensystems geeignet sind und modulatorische Aktivität an Quisqualat-Rezeptoren besitzen.

Es wurde nun gefunden, daß 8-Alkoxy-substituierte 2,3-Benzodiazepinderivate sich gegenüber den bekannten Verbindungen durch bessere Eigenschaften auszeichnen.

Die Erfindung betrifft die Verbindungen der Formel I 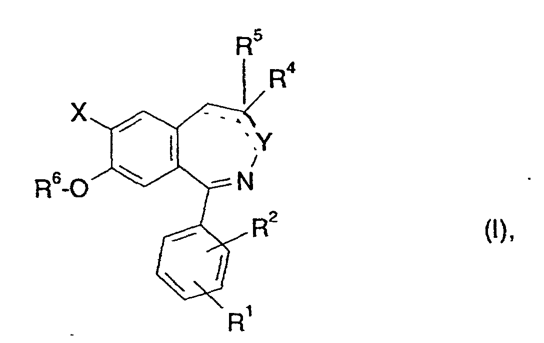 worin
X Wasserstoff oder Halogen,
Y -NR³- oder -N=,
R¹ und R² gleich oder verschieden sind und Wasserstoff, C₁ - C₆-Alkyl, Nitro, Halogen, die Gruppe -NR⁸R⁹, -O-C₁₋₄-Alkyl, -CF₃, -OH, C₁₋₆-Alkanoyloxy,
R³ Wasserstoff, die Gruppe -CO-R¹⁰, C₁₋₆-Alkyl oder C₃₋₇-Cycloalkyl,
R⁴ gegebenenfalls mit C₁₋₆-Alkoxy und Halogen substituiertes C₁ - C₆-Alkyl,
R⁵ Wasserstoff oder
R⁴ und R⁵ gemeinsam Sauerstoff,
R⁶ C₁₋₄-Alkyl,
R⁸ und R⁹ gleich oder verschieden sind und Wasserstoff, C₁ - C₆-Alkyl oder -CO-C₁₋₆-Alkyl,
R¹⁰ Wasserstoff, gegebenenfalls mit C₁₋₆-Alkoxy und Halogen substituiertes C₁ - C₆-Alkyl, gegebenenfalls mit C₁₋₆-Alkoxy und Halogen substituiertes C₆₋₁₀- Aryl, die Gruppe -NR¹¹R¹², -O-C₁₋₆-Alkyl, C₃₋₇-Cycloalkyl, C₂₋₆-Alkenyl oder -O-C₃₋₇-Cycloalkyl,
R¹¹ und R¹² gleich oder verschieden sind und Wasserstoff, gegebenenfalls mit
   C₁₋₆-Alkoxy und Halogen substituiertes C₁ - C₆-Alkyl oder gegebenenfalls mit
   C₁₋₆Alkoxy und Halogen substituiertes C₆₋₁₀-Aryl und
   ―C···C··· eine Doppelbindung oder Einfachbindungen
   bedeuten sowie deren Isomere und physiologisch verträglichen Salze, mit Ausnahme von 1-(3-Chlorphenyl)-4-methyl-8-methoxy-5H-2,3-benzodiazepin.

Unter Alkyl ist jeweils ein geradkettiger oder verzweigter Alkylrest wie beispielsweise Methyl, Ethyl, Propyl, Isopropyl, Butyl, Isobutyl, sek. Butyl, tert. Butyl, Pentyl, Isopentyl oder Hexyl zu verstehen. Als Substituenten des Alkylrestes seien C₁ - C₆-Alkoxy oder Halogen genannt.

Liegt ein halogenierter Alkylrest vor, so kann dieser ein- oder mehrfach halogeniert bzw. perhalogeniert sein.

Unter Halogen ist Fluor, Chlor, Brom and Jod zu verstehen.

Als Arylrest sei beispielsweise Naphthyl und bevorzugt Phenyl genannt. Der Substituent ist beispielsweise C₁₋₆-Alkoxy oder Halogen.

Unter Cycloalkyl ist jeweils Cyclopropyl, Cyclobutyl, Cyclopentyl, Cyclohexyl und Cycloheptyl gemeint, insbesondere C₃₋₅-Cycloalkyl.

Die Alkenylreste können geradkettig oder verzweigt sein. Beispielsweise seien genannt: 1-Propenyl, 2-Propenyl, 3-Methyl-2-propenyl, 1-Butenyl, 2-Butenyl, Methallyl, Vinyl.

Als Alkanoylreste sind geradkettige oder verzweigte aliphatische Carbonsäurereste geeignet wie Formyl, Acetyl, Propionyl, Butanoyl, Isopropylcarbonyl, Caproyl, Valeroyl, Trimethylacetyl u.a..

Die physiologisch verträglichen Salze leiten sich von anorganischen und organischen Säuren ab. Geeignet sind anorganische Säuren wie beispielsweise Halogenwassertoffsäuren wie Chlorwasserstoffsäure, Bromwasserstoffsäure, Phosphorsäure, Schwefelsäure oder organische Säuren wie beispielsweise aliphatische oder aromatische Mono- oder Dicarbonsäuren wie Ameisensäure, Essigsäure, Maleinsäure, Fumarsäure, Succinsäure, Milchsäure, Weinsäure, Zitronensäure, Oxalsäure, Glyoxylsäure oder Sulfonsäuren, beispielsweise C₁₋₄-Alkansulfonsäuren wie Methansulfonsäure oder gegebenenfalls durch Halogen oder C₁₋₄-Alkyl substituierte Benzolsulfonsäuren wie p-Toluolsulfonsäure.

Die Verbindungen der Formel I umfassen auch die möglichen tautomeren Formen, die E-oder Z-Isomeren oder, falls chirale Zentren vorhanden sind, die Diastereomeren und deren Gemische und die Razemate und Enantiomeren.

Bevorzugte Verbindungen der allgemeinen Formel I sind die, in der R¹ Amino oder Nitro und R² Wasserstoff bedeutet.

Die Verbindungen der allgemeinen Formel I sowie deren physiologisch verträglichen Salze sind aufgrund ihrer nichtkompetitiven Hemmung der AMPA-Rezeptoren als Arzneimittel verwendbar. Aufgrund ihres Wirkprofils eignen sich die erfindungsgemäßen Verbindungen zur Behandlung von Krankheiten, die durch Hyperaktivität excitatorischer Aminosäuren wie zum Beispiel Glutamat oder Aspartat hervorgerufen werden. Da die neuen Verbindungen als nichtkompetitive Antagonisten excitatorischer Aminosäuren wirken, eignen sie sich insbesondere zur Behandlung von solchen Krankheiten, die über die Rezeptoren excitatorischer Aminosäuren, insbesondere den AMPA-Rezeptor, beeinflußt werden .

Die pharmakologische Wirksamkeit der Verbindungen der Formel I wurde mittels der nachfolgend beschriebenen Teste bestimmt:

Männliche NMRI Mäuse mit einem Gewicht von 18-22 g wurden unter kontrollierten Verhältnissen (6°°-18°° Uhr Hell/Dunkelrythmus, bei freiem Zugang zu Futter und Wasser) gehalten und ihre Zuordnung zu Gruppen wurde randomisiert. Die Gruppen bestanden aus 5 - 16 Tieren. Die Beobachtung der Tiere wurde zwischen 8°° und 13°° Uhr vorgenommen.

AMPA wurde in den linken Ventrikel von frei beweglichen Mäusen gespritzt. Der Applikator bestand aus einer Kanüle mit einer Vorrichtung aus rostfreiem Stahl, die die Tiefe der Injektion auf 3,2 mm begrenzt. Der Applikator war an eine Injektionspumpe angeschlossen. Die Injektionsnadel wurde perpendicular zu der Oberfläche des Schädels nach den Koordinaten von Montemurro und Dukelow eingeführt. Die Tiere wurden bis zum Auftreten von clonischen bzw. tonischen Krämpfen bis zu 180 sec. beobachtet. Die clonischen Bewegungen, die länger als 5 sec. andauern, wurden als Krämpfe gezählt. Der Anfang der clonischen Krämpfe wurde als Endpunkt für die Bestimmung der Krampfschwelle verwendet. Die Dosis, die notwendig war, um die Krampfschwelle um 50% herauf- bzw. herabzusetzen (THRD₅₀) wurde in 4-5 Experimenten bestimmt. Die THRD₅₀ und die Vertrauensgrenze wurde in einer Regressionsanalyse bestimmt.

Die Ergebnisse dieser Versuche zeigen, daß die Verbindung der Formel I und deren Säureadditionssalze funktionelle Störungen des AMPA-Rezeptors beeinflussen. Sie eignen sich daher zu Herstellung von Arzneimitteln zur symptomatischen und präventiven Behandlung von Erkrankungen, die durch Veränderung der Funktion des AMPA-Rezeptor-Komplexes ausgelöst werden.
Die Behandlung mit den erfindungsgemäßen Verbindungen verhindert bzw. verzögert die infolge der Erkrankung auftretenden Zellschädigungen und funktionellen Störungen und vermindert die dadurch entstehenden Symptome.

Erfindungsgemäß können die Verbindungen verwendet werden zur Behandlung neurologischer und psychiatrischer Störungen, die durch die Überstimulation des AMPA-Rezeptors ausgelöst werden. Zu den neurologischen Erkrankungen , die funktionell und präventiv behandelt werden können, gehören zum Beispiel neurodegenerative Störungen wie Morbus Parkinson, Morbus Alzheimer, Chorea Huntington, Amyotrophe Lateralsklerose und Olivopontocerebelläre Degeneration. Erfindungsgemäss können die Verbindungen zur Prävention des postischämischen Zelluntergangs, des Zelluntergangs nach Hirntrauma, bei Schlaganfall, Hypoxie, Anoxie und Hypoglykämie und zur Behandlung der Senilen Demenz, Aids Demenz, neurologischer Symptome, die mit HIV-Infektionen zusammenhängen, Multiinfarkt Demenz sowie Epilepsie und Muskelspastik verwendet werden. Zu den psychiatrischen Erkrankungen gehören Angstzustände, Schizophrenie, Migräne, Schmerzzustände, sowie die Behandlung von Schlafstörungen und der Entzugssymptomatik nach Drogenmißbrauch wie bei Alkohol-, Kokain-, Benzodiazepin- oder Opiat-Entzug. Die Verbindungen können außerdem eine Anwendung in der Prävention der Toleranzentwicklung während der Langzeitbehandlung mit sedativen Arzneimitteln wie zum Beispiel Benzodiazepinen, Barbituraten und Morphin finden. Darüberhinaus können die Verbindungen als Anästhetika (Narkose), Anti-Schmerzmittel oder Antiemetika benutzt werden.

Zur Verwendung der erfindungsgemäßen Verbindungen als Arzneimittel werden diese in die Form eines pharmazeutischen Präparats gebracht, das neben dem Wirkstoff für die enterale oder parenterale Applikation geeignete pharmazeutische, organische oder anorganische inerte Trägermaterialien, wie zum Beispiel, Wasser, Gelantine, Gummi arabicum, Milchzucker, Stärke, Magnesiumstearat, Talk, pflanzliche Öle, Polyalkylen-glykole usw. enthält. Die pharmazeutischen Präparate können in fester Form, zum Beispiel als Tabletten, Dragees, Suppositorien, Kapseln oder in flüssiger Form, zum Beispiel als Lösungen, Suspensionen oder Emulsionen vorliegen. Gegebenenfalls enthalten sie darüber hinaus Hilfsstoffe wie Konservierungs-, Stabilisierungs-, Netzmittel oder Emulgatoren, Salze zur Veränderung des osmotischen Drucks oder Puffer.

Für die parenterale Anwendung sind insbesondere Injektionslösungen oder Suspensionen, insbesondere wäßrige Lösungen der aktiven Verbindungen in polyhydroxyethoxyliertem Rizinusöl, geeignet.

Als Trägersysteme können auch grenzflächenaktive Hilfsstoffe wie Salze der Gallensäuren oder tierische oder pflanzliche Phospholipide, aber auch Mischungen davon sowie Liposomen oder deren Bestandteile verwendet werden.

Für die orale Anwendung sind insbesondere Tabletten, Dragees oder Kapseln mit Talkum und/oder Kohlenwasserstoffträger oder -binder, wie zum Beispiel Lactose, Mais- oder Kartoffelstärke, geeignet. Die Anwendung kann auch in flüssiger Form erfolgen, wie zum Beispiel als Saft, dem gegebenenfalls ein Süßstoff beigefügt ist.
Die Dosierung der Wirkstoffe kann je nach Verabfolgungsweg, Alter und Gewicht des Patienten, Art und Schwere der zu behandelnden Erkrankung und ähnlichen Faktoren variieren. Die tägliche Dosis beträgt 0,5 - 1000 mg, vorzugsweise 50 - 200 mg, wobei die Dosis als einmal zu verabreichende Einzeldosis oder unterteilt in zwei oder mehreren Tagesdosen gegeben werden kann.

Die Herstellung der erfindungsgemäßen Verbindungen erfolgt beispielsweise dadurch, daß man
a) eine Verbindung der Formel II 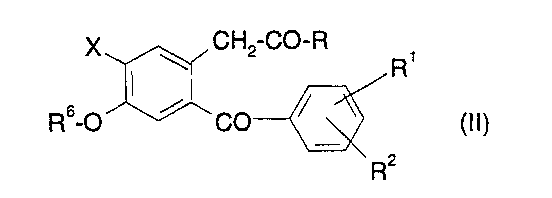 worin R¹, R², R⁶ und X die obige Bedeutung haben und R Hydroxy oder C₁₋₆-Alkyl ist, mit H₂N-NH-R³ cyclisiert oder
b) eine Verbindung der Formel III 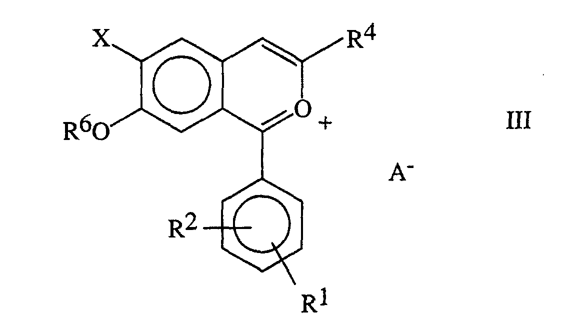
worin R¹, R², R⁴, R⁶ und X die obige Bedeutung haben und A- ein Anion einer anorganischen Base bedeutet, mit H₂N-NHR³ umsetzt und gewünschtenfalls anschließend eine Nitrogruppe und/oder die 3,4-Doppelbindung reduziert und/oder eine Verbindung der allgemeinen Formel I durch Reduktion, Enthalogenierung, Acylierung, Alkylierung, Hydroxylierung, Halogenierung, Einführung einer Carbamoylgruppe oder einer Estergruppe in eine andere Verbindung der allgemeinen Formel I überführt, die Isomeren trennt oder die Salze bildet.

Die Herstellung der Verbindungen der Formel I nach Verfahrensschritt a) erfolgt durch Umsetzung der Diketone der Formel II mit Hydrazinhydrat oder entsprechenden Hydrazinderivaten in polaren Lösungsmitteln wie Alkoholen oder Methylenchlorid in einer Stufe oder ohne Isolierung des Hydrazons zweckmäßigerweise bei erhöhter Temperatur.

Als Anion nach Verfahrensvariante b) sind Halogenide wie Chlorid, Bromid, Jodid, Tetrafluoroborat, Tetrachloroferrat, Hexachlorostannat, Sulfhydrat, Phosphat und Perchlorat geeignet. Man erhält die erfindungsgemäßen Verbindungen vorzugsweise durch Umsetzung des 2-Benzopyrilium-Perchlorates mit Hydrazinhydrat oder Hydrazinderivaten in polaren Lösungsmitteln wie Alkoholen oder Dimethylformamid bei Raumtemperatur oder erhöhter Temperatur.

Die Reduktion in der Nitrogruppe wird in polaren Lösungsmitteln bei Raumtemperatur oder erhöhter Temperatur durchgeführt. Als Katalysatoren für die Reduktion sind Metalle wie Raney-Nickel oder Edelmetall-katalysatoren wie Palladium oder Platin gegebenenfalls auf Trägern geeignet. Statt Wasserstoff können auch zum Beispiel Ammoniumformiat oder Hydrazin in bekannter Weise benutzt werden. Reduktionsmittel wie Zinn-II-chlorid oder Titan-III-chlorid können ebenso verwendet werden wie komplexe Metallhydride eventuell in Gegenwart von Schwermetallsalzen. Als Reduktionsmittel ist auch Eisen nutzbar. Die Reaktion wird dann in Gegenwart einer Säure wie z.B. Essigsäure oder Ammoniumchlorid gegebenenfalls unter Zusatz eines Lösungsmittels wie zum Beispiel Wasser oder Methanol durchgeführt. Wird für die Reduktion Raney-Nickel verwendet, so findet in der Regel keine Enthalogenierung des Substiuenten X statt. Wird eine gleichzeitige Enthalogenierung und Reduktion der Nitrogruppe gewünscht, so ist es zweckmässig Edelmetallkatalysatoren wie Palladium oder Platin und ein säurebindendes Mittel wie z.B. Kaliumcarbonat zu benützen. Als Wasserstoffquelle kann Wasserstoffgas oder z.B. Hydrazinhydrat benutzt werden. Im letzteren Fall ist es zweckmässig die Reaktion bei erhöhter Temperatur durchzuführen.

Die Acylierung kann mit oder ohne Lösungsmittel bei Raumtemperatur oder erhöhter Temperatur mit den üblichen Acylierungsmitteln durchgeführt werden. Als Acylierungsmittel sind Anhydride oder Säurehalogenide geeignet. Als Anhydride können gemischte oder auch symmetrische Anhydride eingesetzt werden. Carbamoylderivate erhält man zweckmässigerweise durch Acylieren mit einem ensprechenden Isocyanat. Wird die Acylierung mit Chlorameisensäureestern wie Chlorameisensäurephenylester vorgenommen, so erhält man durch nachfolgende Reaktion mit primären und sekundären organischen Aminen wie Methylamin die entsprechenden Carbamoylverbindungen bzw. kann durch Umsetzung mit Alkoholen wie Methanol, Ethanol in Gegenwart von katalytischen Mengen NaCN oder mit Titantetraisopropylat in Gegenwart, des für die Umesterung gewünschten Alkohols die entsprechende Estergruppe eingeführt werden.

Wird eine Alkylierung einer Aminogruppe gewünscht, so kann nach üblichen Methoden - beispielsweise mit Alkylhalogeniden - oder nach der Mitsonubo Variante durch Umsetzung mit einem Alkohol in Gegenwart von Triphenylphosphin und Azodicarbon-säureester alkyliert werden, oder man unterwirft das Amin einer reduktiven Aminierung mit Aldehyden oder Ketonen gegebenenfalls nacheinander mit zwei verschiedenen Carbonylverbindungen, wobei man gemischte Derivate erhält [Literatur z.B. Verardo et al. Synthesis (1993), 121; Synthesis (1991), 447; Kawaguchi, Synthesis (1985), 701; Micovic et al. Synthesis (1991), 1043].

Die Acylierung einer Aminogruppe erfolgt in üblicher Weise beispielsweise mit einem Säurehalogenid oder Säureanhydrid gegebenenfalls in Gegenwart einer Base wie Dimethylaminopyridin in Lösungsmitteln wie Methylenchlorid, Tetrahydrofuran oder Pyridin, nach der Schotten-Baumann-Variante in wäßriger Lösung bei schwach alkalischem pH-Wert oder durch Umsetzung mit einem Anhydrid in Eisessig.

Die Einführung der Halogene Chlor, Brom oder Jod über die Aminogruppe kann beispielsweise auch nach Sandmeyer erfolgen, indem man die mit Nitriten intermediär gebildete Diazoniumsalze mit Kupfer(I)chlorid oder Kupfer(I)bromid in Gegenwart der entsprechenden Säure wie Salzsäure oder Bromwasserstoffsäure oder mit Kaliumjodid umsetzt.

Statt der Diazoniumsalze können gegebenenfalls auch die Triazene eingesetzt werden. Wenn ein organischer Salpetrigsäureester benutzt wird, kann man die Halogene z.B. durch Zusatz von Methylenjodid oder Tetrabrommethan einführen in einem Lösungsmittel wie zum Beispiel Dimethylformamid. Die Entfernung der Aminogruppe kann entweder durch Umsetzung mit einem organischen Salpetrigsäureester in Tetrahydrofuran oder durch Diazotierung und reduktive Verkochung des Diazoniumsalzes beispielsweise mit phosphoriger Säure gegebenenfalls unter Zugabe von Kupfer (I) oxid bewerkstelligt werden. Die Einführung von Fluor gelingt beispielsweise durch Balz Schiemann-Reaktion des Diazoniumtetrafluorborates oder nach J. Fluor. Chem. **76**,1996,59-62 durch Diazotierung in Gegenwart von HFxPyridin und anschliessende Verkochung gegebenenfalls in Gegenwart einer Fluoridionenquelle wie z.B. Tetrabutylammoniumfluorid..

Der Ersatz der Amino- durch die Hydroxygruppe erfolgt nach literaturbekannten Methoden vorzugsweise durch Überführung in das Triazen und anschliessende Behandlung mit einem stark sauren Ionenaustauscher (nach Tetr. Letters 1990, 4409 E, J.-R. Barrio et al. J. Chem. Soc. Chem. Commun., 443 (1983)).

Die Isomerengemische können nach den üblichen Methoden wie beispielsweise Kristallisation, Chromatographie oder Salzbildung in die Enantiomere bzw. E/Z-Isomeren aufgetrennt werden.

Die Herstellung der Salze erfolgt in üblicher Weise, indem man eine Lösung der Verbindung der Formel I mit der äquivalenten Menge oder einem Überschuß einer Säure, die gegebenenfalls in Lösung ist, versetzt und den Niederschlag abtrennt oder in üblicher Weise die Lösung aufarbeitet.

Soweit die Herstellung der Ausgangsverbindungen nicht beschrieben wird, sind diese bekannt oder sie erfolgt analog zu bekannten Verbindungen. Die neuen Verbindungen der Formel I sind auch als Zwischenprodukte zur Herstellung pharmakologisch aktiver Verbindung geeignet.

Die nachfolgenden Beispiele erläutern die Herstellung der erfindungsgemäßen Verbindungen.

### Beispiel 1

### 1-(4-Aminophenyl)-8-methoxy-4,5-dihydro-3H-2,3-benzodiazepin-4-on

### Schritt A

Zu einer Suspension von 8,30 g (75.0 mmol) gepulvertem Calciumchlorid in wasserfreiem THF werden bei 0 °C 5,0 g (130.0 mmol) Natrimborhydrid gegeben und nach 30 Minuten Rühren wird eine Lösung von 10,75 g (39.35 mmol) 3-Brom-4-methoxyphenylessigsäuremethylester (O.N.Tolkachev u.a., Zh. Obshsh. Khim. 31 (1961), 1540-1545) in 75 ml THF zugetropft. Anschließend wird das Gemisch 1 Stunde bie 0°C, dann 20 Stunden bei Raumtemperatur gerührt und erneut auf 0° C abgekühlt. Es werden 100 ml Wasser und 100 ml 1n HCl zugegeben und das THF wird abgezogen. Der verbleibende Rest wird mit Essigester extrahiert, und die organische Phase mit Wasser gewaschen, getrocknet und eingedampft.
Es werden so 9,10 g (100%) 2-(3-Brom-4-methoxy-phenyl)-ethanol als Rohprodukt erhalten, das in dieser Form im nächsten Schritt verarbeitet wird.

### Schritt B

In eine Suspension von 7,50 g (32.4 mmol) 2-(3-Brom-4-methoxy-phenyl)-ethanol, 4,90 g (32,4 mmol) 4-Nitro-benzaldehyd und 4,39 g (32.4 mmol) frisch geschmolzenem Zinkchlorid in wasserfreiem Benzol (96 ml) wird 5 Stunden lang trockene Chlorwasserstoff-Gas eingeleitet. Das Reaktionsgemisch wird anschließend mit Essigester verdünnt und mit Wasser neutral gewaschen. Nach dem Trocknen und Eindampfen wird der feste Rückstand aus Essigester (25 ml) umkristallisiert. Man erhält 7,91 g (67%) 6-Brom-7-methoxy-1-(4-nitrophenyl)-isochroman, Schmelzpunkt 144 - 147 °C.

### Schritt C

5,46 g (15.0 mmol) der Verbindung aus Schritt B werden in Aceton (110 ml) mit 22,5 ml Jones-Reagenz über Nacht gerührt. Das ausgeschiedene Salz wird filtriert und mit Aceton nachgewaschen. Das Salz wird dann mit 4 ml Isopropanol 30 Minuten lang gerührt und erneut abfiltriert. Die vereinigten organischen Filtrate werden eingedampft. Der Rückstand wird anschließend in Essigester gelöst, mit Wasser neutral gewaschen und die organische Phase wird getrocknet und eingedampft. Der kristaline Rückstand wird aus Essigsäure (12 ml) umkristallisiert. Man erhält 4,30 g (73%) 5-Brom-4-methoxy-2-(4-nitrobenzoyl)-phenylessigsäure vom Schmelzpunkt 189 - 192 °C.

### Schritt D

3,94 g (10.0 mmol) der Substanz aus Schritt C werden in 75 ml Ethanol gelöst. Nach der Zugabe von 1,50 ml (30 mmol) 98% Hydrazinhydrat wird das Gemisch 5 Stunden zum Sieden erhitzt. Nach dem Abkühlen wird die Lösung mit 20 ml 1n HCl angesäuert und im Vakuum eingeengt. Der Rückstand wird in 8 ml Wasser suspendiert und das kristalline Hydrazon wird mit Wasser gewaschen und im Vakuum getrocknet.
Das Hydrazon wird anschließend in trockenem Methylenchlorid suspendiert und mit einer Lösung von 2,06 g (10,0 mmol) N,N'-Dicyclohexylcarbodiimid in 30 ml Methylenchlorid versetzt und 16 - 20 Stunden gerührt. Das Produkt wird abgesaugt, dann mit Ethanol (24 ml) 10 - 15 Minuten lang zum Sieden erhitzt und erneut filtriert. Es werden so 2,56 g (66%) 7-Brom-8-methoxy-1-(4-nitrophenyl)-4,5-dihydro-3H-2,3-benzodiazepin-4-on vom Schmelzpunkt 245- 256 °C erhalten.

In analoger Weise wird hergestellt:
7-Brom-8-methoxy-1-phenyl-4,5-dihydro-3H-2,3-benzodiazepin-4-on

### Schritt E

1,10 g (2.82 mmol) der vorher beschriebenen Verbindung werden in 40 ml Methylenchlorid und 50 ml Methanol gelöst und nach der Zugabe von 0,39 g (2.82 mmol) Kaliumcarbonat und 0,20 g 10% Palladium/Akivkohle hydriert. Anschließend wird vom Katalysator abgesaugt und das Filtrat eingedampft. Den Rückstand löst man in Chloroform und wäscht mehrmals mit Wasser. Nach wiederholtem Eindampfen wird das Produkt mit Ethanol zum Sieden erhitzt und filtriert. Man erhält 0,62 g (78%) 1-(4-Aminophenyl)-8-methoxy-4,5-dihydro-3H-2,3-benzodiazepin-4-on, Schmelzpunkt 167 - 170 °C.

In analoger Weise wird hergestellt aus der entsprechenden 7-Brom-Verbindung
8-Methoxy-1-phenyl-4,5-dihydro-3H-2,3-benzodiazepin-4-on

### Beispiel 2

### 1-(4-Aminophenyl)-7-chlor-8-methoxy-4,5-dihydro-3H-2,3-benzodiazepin-4-on

### Schritt A

In eine Suspension von 15,70 g (72.0 mmol) 2-(3-Chlor-4-methoxyphenyl)-ethanol (L.S. Fosdick u.a., J. Am. Chem. Soc. **68** (1946), 840-843), 10,23 g (72.0 mmol) Nitrobenzaldehyd und 9,18g (72mmol) frisch geschmolzenem Zinkchlorid in wasserfreiem Benzol (200ml) wird 7 Stunden lang trockenes Chlorwasserstoffgas eingeleitet. Anschließend wird das Reaktionsgemisch mit Essigester verdünnt und mit Wasser neutral gewaschen, getrocknet und eingedampft. Der Rückstand wird aus Essigester (60 ml) umkristallisiert. Man erhält so 10,4 g (45%) 6-Chlor-7-methoxy-1-(4-nitrophenyl)-Isochroman, Schmelzpunkt 150 - 153 °C.

### Schritt B

10,23 g (32.0 mmol) der Substanz des vorhergehenden Schrittes werden mit 55 ml Jones-Reagenz versetzt. Man verfährt wie im Schritt C des Beispiels 1 und das erhaltene Rohprodukt wird aus Essigsäure umkristallisiert. Es werden so 7,28 g (68%) 5-Chlor-4-methoxy-2-(4-nitrobenzoyl)-phenylessigsäure vom Schmelzpunkt 185 - 188 °C erhalten.

### Schritt C

5,32 g (16.0 mmol) der obigen Verbindung wer den in 120 ml Ethanol mit 2,40 ml (50 mmol) 98% Hydrazinhydrat versetzt und 5 Stunden zum Sieden erhitzt. Anschliessend wird wie im Schritt D des Beispiels 1 verfahren. Das Rohrprodukt wird durch mehrmaliges Erhitzen in Ethanol gereinigt. Man erhält 3,48 g (63%) 7-Chlor-8-methoxy-1-(4-nitrophenyl)-4,5-dihydro-3H-2,3-benzodiazepin-4-on vom Schmelzpunkt 258 - 262 °C.

### Schritt D

0,48 g (1.4 mmol) der Verbindung des vorangehenden Schrittes werden in 10 ml DMF und 10 ml Methanol gelöst und in Gegenwart von Ra/Ni Katalysator 0,27 ml (5.5 mmol) 98% Hydrazinhydrat werden zugegeben. Das Gemisch wird danach 2 Stunden lang gerührt. Es wird vom Katalysator abgesaugt und das Filtrat wird im Vakuum eingedampft. Man suspendiert den Rückstand in Wasser und filtriert das Produkt. Es werden so 0,31 g (70%) 1-(4-Aminophenyl)-7-chlor-8-methoxy-4,5-dihydro-3H-2,3-benzodiazepin-4-on erhalten, Schmelzpunkt 234 - 236 °C.

### Beispiel 3

### 1-(4-Aminophenyl)-4-methyl-8-methoxy-5H-2,3-benzodiazepin

### Schritt A

Zu einer Lösung von 32,46 g (132.4 mmol) 1-(3-Brom-4-methoxyphenyl)-propan-2-ol und 20.01 g (132.4 mmol) 4-Nitro-benzaldehyd in 166 ml trockenem Benzol werden 18,04 g (132.4 mmol) frisch geschmolzenes Zinkchlorid gegeben und anschließend trockenes Chlorwasserstoffgas 3 Stunden lang eingeleitet. Das Reaktionsgemisch wird dann 1 Stunde zum Sieden erhitzt und mit 150 ml Wasser verrührt. Die organische Phase wird abgetrennt und nacheinander mit Wasser (2 x 50 ml), 20% Natriumhydrogensulfit Lösung (100 ml), 8% Natriumbicarbonat Lösung (50 ml) und Wasser (2 x 50 ml) gewaschen und getrocknet. Nach dem Eindampfen erhält man ein dickes Öl, das beim Behandeln mit heißem Ethanol (450 ml) kristallines 6-Brom-3-methyl-7-methoxy-1-(4-nitrophenyl)-isochroman ergibt: 20.39 g (41%), Schmelzpunkt 128- 129 °C.

### Schritt B

Zu einer Lösung von 20,39 g (53,9 mmol) der Verbindung aus Schritt A in 250 ml Aceton werden bei 20 °C während ca. 20 Minuten 70,78 ml (188.6 mmol) Jones Reagenz getropft. Das Rektionsgemisch wird dann 4 Stunden weitergerührt und anschließend mit Wasser (750 ml) versetzt. Die ausgefallene Substanz wird abgesaugt und mit Wasser gewaschen. Das erhaltene Rohprodukt (20.32 g) wird in Essigester (203 ml) gelöst und mit 4,9 ml (56.3 mmol) 70% Perchlorsäure versetzt. Man erwärmt die Lösung für einige Minuten zum Sieden, wobei sich Kristalle auszuscheiden beginnen. Nach dem Abkühlen wird das Produkt abgesaugt und mit Essigester gewaschen. Man erhält 11,26 g (44%) 6-Brom-3-methyl-7-methoxy-1-(4-nitrophenyl)-2-benzopyriliumperchlorat vom Schmelzpunkt 252 - 253 °C (Zers.)

### Schritt C

4,08 g (8.59 mmol) des 2-Benzopyrilium Salzes aus Schritt B werden in DMF (20 ml) gelöst und mit 1,29 ml (25.7 mmol) 98% Hydrazinhydrat versetzt. Nach Rühren bei 25 °C für 15 Minuten wird das Produkt mit 80 ml Wasser ausgefällt. Nach Absaugen und Waschen mit Wasser erhält man 3,32 g 7-Brom-8-methoxy-4-methyl-1(4-nitrophenyl)-5H-2,3-benzodiazepin als Rohprodukt, Schmelzpunkt 218 - 221 °C (Zers.) Nach einmaligem Umkristallisieren aus 15 ml Ethanol werden 2,96 g (89%) des Produktes in Form von gelben Kristallen mit dem Schmelzpunkt 234 - 236 °C (Zers.) erhalten.

In grundsätzlich analoger Weise wird hergestellt über die entsprechenden Stufen A - C
7-Brom-8-methoxy-4-methyl-1-phenyl-5H-2,3-benzodiazepin

### Schritt D

0,50 g (1.28 mmol) des Benzodiazepin-Derivates aus dem vorhergehenden Schritt C werden in Methylcellosolv (50 ml) suspendiert. Nach Zugabe von 0,27 g (1.89 mmol) trockenem Kaliumcarbonat, 0,50-g Palladium auf Kohle (10%) und 0,2ml Hydrazinhydrat (98%) wird das Gemisch 1 Stunde bei 100 °C gerührt. Man saugt vom Feststoff ab und dampft das Filtrat ein. der ölige Rückstand wird mit Wasser zum Kristallisieren gebracht. Man kristallisiert das Rohprodukt aus Ethanol (3,5 ml) um, wobei man 0,21 g (59%) der Titelverbindung in Form von beinahe weißen Kristallen mit Schmelzpunkt 136 - 138 °C erhält.

In analoger Weise wird hergestellt:
Aus der entsprechenden 7-Brom-Verbindung
8-Methoxy-4-methyl-1-phenyl-5H-2,3-benzodiazepin

### Beispiel 4

### 3-Acetyl-1-(4-aminophenyl)-4-methyl-8-methoxy-4,5-dihydro-3H-2,3-benzodiazepin

### Schritt A

Zu einer Suspension von 1,4 g (3.6 mmol) 7-Brom-4-methyl-8-methoxy-1-(4-nitrophenyl)-5H-2,3-benzodiazepin (Beispiel 3, Schritt C) in 60 ml Methanol werden zuerst 3,5 ml (43.2 mmol) konz. Salzsäure und anschließend während ca. 10 Minuten portionsweise 1,80 g (47.5 mmol) Natriumborhydrid gegeben. Die Suspension wird 1 Stunde weiter gerührt, und dann mit Wasser (60 ml) verdünnt. Das kristalline Material wird abgesaugt und mit 50 % wässrigem Methanol (3 x 50 ml) gewaschen. Das als Rohprodukt erhaltene 7-Brom-4-methyl-8-methoxy-1-(4-nitrophenyl)-4,5-dihydro-3H-2,3-benzodiazepin wird durch Suspendieren in 8 ml heißem Ethanol weiter gereinigt. Man erhält nach dem Absaugen 1,23 g (88%) zitronengelbe Kristalle vom Schmelzpunkt 172-174 °C.

In analoger Weise wird hergestellt:
7-Brom-4-methyl-8-methoxy-1-phenyl-4,5-dihydro-3H-2,3-benzodiazepin

### Schritt B

0,60 g (1.5 mmol) der Dihydro-Verbindung aus dem vorangegangenen Schritt A- werden in 3 ml Essigsäuranhydrid gelöst. Nach einer Stunde Reaktionszeit bei 25 °C wird die Lösung mit 15 ml Wasser verrührt und die ausgefallenen Kristalle werden abgesaugt und mit Wasser gewaschen (4 x 5 ml). Das als Rohprodukt isolierte 3-Acetyl-7-brom-4-methyl-8-methoxy-1-(4-nitrophenyl)-4,5-dihydro-3H-2,3-benzodiazepin ergibt nach dem Umkristallisieren aus Ethanol (32 ml) 0,56 g (86%) reines Produkt in Form von gelben Kristallen mit Schmelzpunkt 194 - 196 °C.

In analoger Weise wird hergestellt:
3-Acetyl-7-brom-4-methyl-8-methoxy-1-phenyl-4,5-dihydro-3H-2,3-benzodiazepin

### Schritt C

0,54 g (1.2 mmol) des Acetyl-Derivates vom vorherigen Schritt B wird gemäß Beispiel 3, Schritt D reduziert. Man erhält 0,36 g der Titelverbindung als Rohprodukt. Weitere Reinigung erfolgt durch Säulenchromatographie an Kieselgel mit dem Elutionsmittel Chloroform:Methanol = 95:5. Umkristallisieren des Produktes aus 12 ml Ethanol liefert 0,16 g (67%) der Titelverbindung als gelbe Kristalle, Schmelzpunkt 237 - 238 °C.

In grundsätzlich analoger Weise werden hergestellt aus den entsprechenden 7-Brom-Verbindungen:
3-Methoxycarbonyl-1-(4-aminophenyl)-4-methyl-8-methoxy-4,5-dihydro-3H-2,3-benzodiazepin
3-Acetyl-4-methyl-8-methoxy-1-phenyl-4,5-dihydro-3H-2,3-benzodiazepin

### Beispiel 5

### 1-(4-Aminophenyl)-4-methyl-3-methylcarbamoyl-8-methoxy-4,5-dihydro-3H-2,3-benzodiazepin

### Schritt A

0,60 g (1.5 mmol) 7-Brom-4-methyl-8-methoxy-1-(4-nitrophenyl)-4,5-dihydro-3H-2,3-benzodiazepin (Beispiel 4, Schritt A) werden in 12 ml trockenem Methylenchlorid gelöst und mit 0,64 ml (10.5 mmol) Methylisocyanat versetzt. Nach einer Reaktionsdauer von 9 Tagen bei 25 °C wird die Lösung eingedampft und der Rückstand aus Ethanol (10 ml) umkristallisiert. Man erhält 0,56 g (83%) 7-Brom-4-methyl-3-methylcarbamoyl-8-methoxy-1-(4-nitrophenyl)-4,5-dihydro-3H-2,3-benzodiazepin in Form von gelben Kristallen vom Schmelzpunkt 224 - 226 °C.

In analoger Weise wird hergestellt:
7-Brom-4-methyl-3-methylcarbamoyl-8-methoxy-1-phenyl-4,5-dihydro-3H-2,3-benzodiazepin

### Schritt B

0,54 g (1.2 mmol) der Nitro-Verbindung aus dem obigen Schritt A werden gemäß Beispiel 3, Schritt D reduziert. Man erhält 0,32 g der Titelverbindung als Rohprodukt, das durch Säulenchromatographie weiter gereinigt wird (Kieselgel, Elutionsmittel Essigester:Benzol = 4:1). Nach dem Eindampfen der Fraktionen wird das Produkt aus Essigester umkristallisiert und man erhält 0,20 g (49%) der reinen Titelverbindung vom Schmelzpunkt 190 - 191 °C.

In analoger Weise wird hergestellt:
8-Methoxy-4-methyl-3-methylcarbamoyl-1-phenyl-4,5-dihydro-3H-2,3-benzodiazepin

### Beispiel 6

### 1-(4-Aminophenyl)-7-chlor-4-methyl-8-methoxy-5H-2,3-benzodiazepin

### Schritt A

Eine Lösung von 21,16 g (105.4 mmol) 1-(3-Chlor-4-methoxyphenyl)-2-hydroxypropan in 106 ml trockenem Benzol wird mit 15,93 g (105,4 mmol) 4-Nitrobenzaldehyd und 14,36 g (105.4 mmol) frisch geschmolzenem Zinkchlorid versetzt und trockenes Chlorwasserstoffgas wird 3 Stunden eingeleitet. Anschließend wird das Gemisch 30 Minuten zum Sieden erhitzt. Aufarbeitung erfolgt nach dem Verfahren, das in Beispiel 3, Schritt A beschrieben ist. Das erhaltene 6-Chlor-3-methyl-7-methoxy-1-(4-nitrophenyl)-isochroman wird zuerst als ein öliges Rohprodukt isoliert, das aber beim Behandeln mit 370 ml heißem Ethanol kristallisiert:
15,79 g (50%, Schmelzpunkt 118 - 120 °C.

### Schritt B

16,75 g (50.1 mmol) des Isochroman-Derivates aus dem vorangehenden Schritt A werden gemäß dem Verfahren des Schrittes B vom Beispiel 3 nach Jones oxydiert. Man behandelt das Rohprodukt in 165 ml heißem Essigester mit 4,56 ml (52.47 mmol) 70% Perchlorsäure und erhält 12,09 g rohes 6-Chlor-3-methyl-7-methoxy-1-(4-nitrophenyl)-2-benzopyriliumperchlorat, das nach einem Suspendieren in heißem Eisessig (60 ml) 10,25 g (48%) gelbe Kristalle des reinen Produktes mit Schmelzpunkt 244 - 246 °C ergibt.

### Schritt C

Zu einer Suspension von 10,15 g (23.8 mmol) des Perchlorat-Salzes des vorangehenden Schrittes B in 103 ml Isopropanol werden 2,74 ml (54.7 mmol) Hydrazinhydrat gegeben. Das Reaktionsgemisch wird 30 Minuten zum Sieden erhitzt und nach dem Abkühlen saugt man das ausgefallene Produkt ab und wäscht es mit Isopropanol. Das erhaltene rohe 7-Chlor-4-methyl-8-methoxy-1-(4-nitrophenyl)-5H-2,3-benzodiazepin (6,13 g) wird durch Rühren in 60 ml siedendem Wasser weiter gereinigt. Man erhält 4,71 g (58 %) gelbe Kristalle vom Schmelzpunkt 234 - 236 °C.

### Schritt D

Eine Suspension von 0,24 g (0.7 mmol) des Benzodiazepins aus Schritt C in 24 ml Methanol wird mit Ra/Ni Katalysator und 0.075ml (1.54 mmol) 98% Hydrazinhydrat versetzt. Anschließend wird das Gemisch 1 Stunde nachgerührt, wobei heftige Wasserstoff-Entwicklung eintritt. Man saugt vom Katalysator ab und dampft das Filtrat ein. Das resultierende Öl wird durch Anreiben mit Wasser zum Kristallisieren gebracht. Nach dem Umkristallisieren aus Ethanol (4ml) erhält man 0,16 g (73%) der Titelverbindung vom Schmelzpunkt 198 - 200°C.

### Beispiel 7

### 3-Cyclopropylcarbonyl-1-(4-aminophenyl)-4-methyl-8-methoxy-3H-2,3-benzodiazepin

### Schritt A

0,5g 7-Brom-1-(4-nitrophenyl)-4-methyl-8-methoxy-5H-2,3-benzodiazepin (Beispiel 3, Schritt C) werden in 15ml Benzol suspendiert wird mit 0,53g Kaliumcarbonat und 0,17ml Cyclopropylcarbonsäurechlorid versetzt und anschliessend 5 Stunden zum Rückfluss erhitzt. Anschliessend wird das Gemisch mit Wasser verrührt, die organische Phase abgetrennt, nacheinander mit Sodalösung und Wasser gewaschen, getrocknet, filtriert und eingeengt. Der Rückstand wird durch Ausrühren in Ethanol gereinigt und man erhält 0,38g 7-Brom-3-cyclopropylcarbonyl-1-(4-nitrophenyl)-4-methyl-8-methoxy-3H-2,3-benzodiazepin der Titelverbindung vom Schmelzpunkt.231-233°C.

### Schritt B

0,38mg (0,83mmol) der Nitroverbindung aus Schritt A werden gemäss Beispiel 2, Schritt D reduziert. Man erhält 0,31g (87%) 1-(4-Aminophenyl)-7-brom-3-cyclopropylcarbonyl-4-methyl-8-methoxy-3H-2,3-benzodiazepin vom Schmelzpunkt 224-226°C.

### Schritt C

0,31g (0,73mmol) der Aminophenylverbindung aus Schritt C werden in 50ml Methanol gelöst und nach der Zugabe von 0,31g Palladium auf Kohle (10%) und 0,20g (1,45mmol) Kaliumcarbonat wird drei Tade lang katalytisch hydriert. Nach dem Absaugen vom Katalysator wird eingedampft und der Rückstand mit Wasser verrieben. Das erhaltene Rohprodukt wird durch Säulenchromatographie an Kieselgel mit Essigester:Benzol=1:1 als Elutionsmittel gereinigt. Man erhält 0,14g der Titelverbindung vom Schmelzpunkt 214-216 °C.

In analoger Weise werden hergestellt:
3-Acetyl-1-(4-aminophenyl)-4-methyl-8-methoxy-3H-2,3-benzodiazepin
3-n-Propionyl-1-(4-aminophenyl)-4-methyl-8-methoxy-3H-2,3-benzodiazepin
3-Acetyl-8-methoxy-4-methyl-1-phenyl-3H-2,3-benzodiazepin
3-Cyclopropylcarbonyl-8-methoxy-4-methyl-1-phenyl-3H-2,3-benzodiazepin

### Beispiel 8

### 1-(4-Aminophenyl)-8-methoxy-4-methyl-3-propionyl-4,5-dihydro-3H-2,3-benzodiazepin

### Schritt A

0,67g (1.7 mmol) 7-Brom-8-methoxy-4-methyl-1-(4-nitrophenyl)-4,5-dihydro-3H-2,3-benzodiazepin (Beispiel 4, Schritt A) werden in 3,35 ml Propionsäureanhydrid suspendiert und 3 Stunden lang bei Raumtemperatur gerührt. Das Gemisch wird mit Wasser verdünnt und das Produkt filtriert. Das Rohprodukt wird nach dem Waschen mit Wasser und Trocknen säulenchromatographisch gereinigt (Kieselgel, Elutionsmittel Benzol:Essigester: 4:0,2). Man erhält 0,53 g (70%) 7-Brom-8-methoxy-4-methyl-1-(4-nitrophenyl)-3-propionyl-3H-2,3-benzodiazepin als dickes Öl.

### Schritt B

0,53 g (1.18 mmol) des Propionyl-Derivates aus Schritt A werden gemäß Beispiel 3, Schritt D reduziert. Man erhält 0,35 g der Titelverbindung als Rohprodukt, das durch Säulenchromatographie weiter gereinigt wird (Kieselgel, Elutionsmittel Essigester:Benzol=4:1). Das Produkt gibt nach einem Umkristallisieren aus Ethanol 0,14 g (35%) reine Titelverbindung vom Schmelzpunkt 166-168°C.

### Beispiel 9

### 1-(4-Aminophenyl)-3-cyclopropylcarbonyl-8-methoxy-4-methyl-4,5-dihydro-3H-2,3-benzodiazepin

### Schritt A

0,69g (1.77 mmol) 7-Brom-8-methoxy-4-methyl-1-(4-nitrophenyl)-4,5-dihydro-3H-2,3-benzodiazepin (Beispiel 4, Schritt A) werden in Methylenchlorid (15 ml) in Gegenwart von 0,29 ml (2.1 mmol) Triethylamin mit 0,19 ml (2.1 mmol) Cyclopropansäurechlorid bei Raumtemperatur während 1,5 Stunden acyliert. Nach dem Eindampfen und Suspendieren mit Wasser erhält man 0,78 g eines Rohproduktes, das durch Suspendieren in heißem Ethanol gereinigt wird. Man erhält 0,75 g (93 %) 7-Brom-3-cyclopropylcarbonyl-8-methoxy-4-methyl-1-(4-nitrophenyl)-4,5-dihydro-3H-2,3-benzodiazepin vom Schmelzpunkt 194-196°C.

### Schritt B

Reduktion und Enthalogenierung erfolgt aus 0,73 g (1,59 mmol) der Verbindung aus Schritt A gemäß Beispiel 3, Schritt D und so erhält man 0,51 g der Titelverbindung als Rohprodukt. Nach Säulenchromatographie (Kieselgel, Elutionsmittel Essigester:Benzol: 4:1) wird das Produkt aus Essigester umkristallisiert und so erhält man 0,36 g (65 %) der Titelverbindung vom Schmelzpunkt 93-95°C.

## Patentansprüche

1. Verbindungen der Formel I 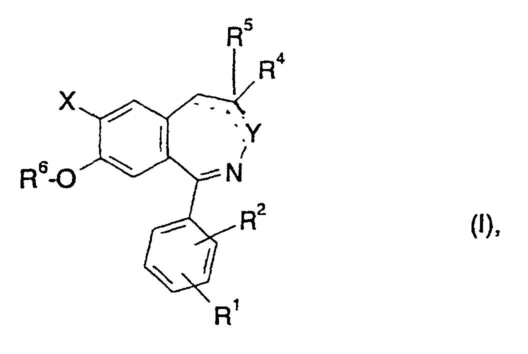 worin
X Wasserstoff oder Halogen,
Y -NR³- oder -N=,
R¹ und R² gleich oder verschieden sind und Wasserstoff, C₁ - C₆-Alkyl, Nitro, Halogen, die Gruppe -NR⁸R⁹, -O-C₁₋₄-Alkyl, CF₃, OH oder C₁₋₆-Alkanoyloxy,
R³ Wasserstoff, die Gruppe -CO-R¹⁰, C₁₋₆-Alkyl oder C₃₋₇-Cycloalkyl,
R⁴ gegebenenfalls mit C₁₋₆-Alkoxy und Halogen substituiertes C₁ - C₆-Alkyl,
R⁵ Wasserstoff oder
R⁴ und R⁵ gemeinsam Sauerstoff,
R⁶ C₁₋₄-Alkyl,
R⁸ und R⁹ gleich oder verschieden sind und Wasserstoff, C₁ - C₆-Alkyl oder -CO-C₁₋₆-Alkyl,
R¹⁰ Wasserstoff, gegebenenfalls mit C₁₋₆-Alkoxy und Halogen substituiertes C₁ - C₆-Alkyl, gegebenenfalls mit C₁₋₆-Alkoxy und Halogen substituiertes C₆₋₁₀- Aryl, die Gruppe -NR¹¹R¹², -O-C₁₋₆-Alkyl, C₃₋₇-Cycloalkyl, C₂₋₆-Alkenyl oder -O-C₃₋₇-Cycloalkyl,
R¹¹ und R¹² gleich oder verschieden sind und Wasserstoff, gegebenenfalls mit C₁₋₆-Alkoxy und Halogen substituiertes C₁ - C₆-Alkyl oder gegebenenfalls mit C₁₋₆-Alkoxy und Halogen substituiertes C₆₋₁₀-Aryl und ―C···C··· eine Doppelbindung oder Einfachbindungen
bedeuten sowie deren Isomere und physiologisch verträglichen Salze, mit Ausnahme von 1-(3-Chlorphenyl)-4-methyl-8-methoxy-5H-2,3-benzodiazepin.

2. 1-(4-Aminophenyl)-8-methoxy-4,5-dihydro-3H-2,3-benzodiazepin-4-on
1-(4-Aminophenyl)-4-methyl-8-methoxy-5H-2,3-benzodiazepin
3-Acetyl-1-(4-aminophenyl)-4-methyl-8-methoxy-4,5-dihydro-3H-2,3-benzodiazepin
3-n-Propionyl-1-(4-aminophenyl)-4-methyl-8-methoxy-4,5-dihydro-3H-2,3-benzodiazepin
3-Cyclopropylcarbonyl-1-(4-aminophenyl)-4-methyl-8-methoxy-4,5-dihydro-3H-2,3-benzodiazepin
3-Methoxycarbonyl-1-(4-aminophenyl)-4-methyl-8-methoxy-4,5-dihydro-3H-2,3-benzodiazepin
3-Methylcarbamoyl-1-(4-aminophenyl)-4-methyl-8-methoxy-4,5-dihydro-3H-2,3-benzodiazepin
3-Acetyl-1-(4-aminophenyl)-4-methyl-8-methoxy-3H-2,3-benzodiazepin
3-n-Propionyl-1-(4-aminophenyl)-4-methyl-8-methoxy-3H-2,3-benzodiazepin
3-Cyclopropylcarbonyl-1-(4-aminophenyl)-4-methyl-8-methoxy-3H-2,3-benzodiazepin
gemäß Anspruch 1.

3. Arzneimittel enthaltend eine Verbindung der Formel I gemäß Anspruch 1 und pharmazeutisch übliche Träger- und Hilfsstoffe.

4. Verfahren zur Herstellung der Verbindungen der Formel I gemäß Anspruch 1 **dadurch gekennzeichnet, daß** man
a) eine Verbindung der Formel II 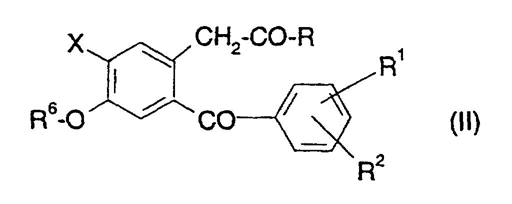 worin R¹, R², R⁶ und X die obige Bedeutung haben und R Hydroxy oder C₁₋₆-Alkyl ist, mit H₂N-NH-R³ cyclisiert oder
b) eine Verbindung der Formel III 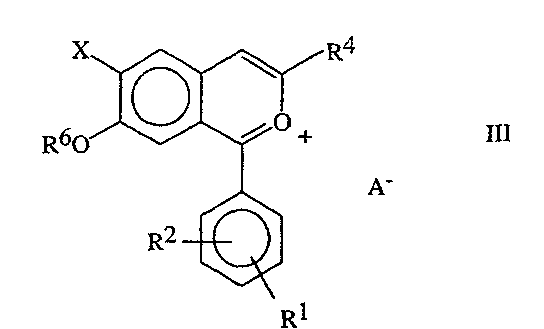
worin R¹, R², R⁴, R⁶ und X die obige Bedeutung haben und A⁻ ein Anion einer anorganischen Base bedeutet, mit H₂N-NHR³ umsetzt und gewünschtenfalls anschließend eine Nitrogruppe und/oder die 3,4-Doppelbindung reduziert und/oder eine Verbindung der allgemeinen Formel I durch Reduktion, Enthalogenierung, Acylierung, Alkylierung, Hydroxylierung, Halogenierung, Einführung einer Carbamoylgruppe oder einer Estergruppe in eine andere Verbindung der allgemeinen Formel I überführt, die Isomeren trennt oder die Salze bildet.

5. Verwendung der Verbindungen nach Anspruch 1 oder 2 zur Herstellung eines Arzneimittels zur Behandlung von Krankheiten, die durch Hyperaktivität excitatorischer Aminosäuren ausgelöst werden.

6. Verwendung nach Anspruch 5 zur symptomatischen und präventiven Behandlung von Erkrankungen, die durch Überstimulation des AMPA-Rezeptors ausgelöst werden.

## Claims

1. Compounds of formula I 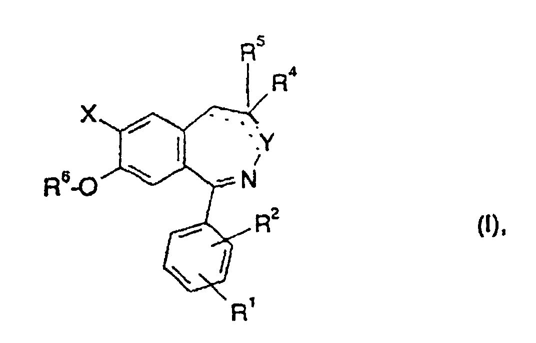 in which
X means hydrogen or halogen,
Y means -NR³- or -N=,
R¹ and R² are the same or different and mean hydrogen, C₁-C₆alkyl, nitro, halogen, the group - NR⁸R⁹,
-O-C₁₋₄alkyl, CF₃, OH or C₁₋₆alkanoyloxy,
R³ means hydrogen, the group -CO-R¹⁰, C₁₋₆alkyl or C₃₋₇cycloalkyl,
R⁴ means C₁-C₆alkyl optionally substituted with C₁₋₆alkoxy and halogen,
R⁵ means hydrogen or
R⁴ and R⁵ together mean oxygen,
R⁶ means C₁₋₄alkyl,
R⁸ and R⁹ are the same or different and mean hydrogen, C₁-C₆alkyl or -CO-C₁₋₆alkyl,
R¹⁰ means hydrogen, C₁-C₆alkyl optionally substituted with C₁-₆alkoxy and halogen, C₆₋₁₀ aryl optionally substituted with C₁-₆alkoxy and halogen, the group -NR¹¹R¹², -O-C₁₋₆alkyl, C₃₋₇cycloalkyl C₂₋ ₆alkenyl or -O-C₃₋₇cycloalkyl,
R¹¹ and R¹² are the same or different and mean hydrogen, C₁-C₆alkyl optionally substituted with C₁-₆alkoxy and halogen or C₆₋₁₀aryl optionally substituted with C₁-₆alkoxy and halogen and
-C...C... means a double bond or single bonds
as well as their isomers and physiologically compatible salts, with the exception of 1-(3-chlorophenyl)-4-methyl-8-methoxy-5H-2,3-benzodiazepine.

2. 1-(4-Aminophenyl)-8-methoxy-4,5-dihydro-3H-2,3-benzodiazepin-4-one
1-(4-aminophenyl)-4-methyl-8-methoxy-5H-2,3-benzodiazepine,
3-acetyl-1-(4-aminophenyl)-4-methyl-8-methoxy-4,5-dihydro-3H-2,3-benzodiazepine,
3-n-propionyl-1-(4-aminophenyl)-4-methyl-8-methoxy-4,5-dihydro-3H-2,3-benzodiazepine,
3-cyclopropylcarbonyl-1-(4-aminophenyl)-4-methyl-8-methoxy-4,5-dihydro-3H-2,3-benzodiazepine,
3-methoxycarbonyl-1-(4-aminophenyl)-4-methyl-8-methoxy-4,5-dihydro-3H-2,3-benzodiazepine,
3-methylcarbamoyl-1-(4-aminophenyl)-4-methyl-8-methoxy-4,5-dihydro-3H-2,3-benzodiazepine,
3-acetyl-1-(4-aminophenyl)-4-methyl-8-methoxy-3H-2,3-benzodiazepine,
3-n-propionyl-1-(4-aminophenyl)-4-methyl-8-methoxy-3H-2,3-benzodiazepine,
3-cyclopropylcarbonyl-1-(4-aminophenyl)-4-methyl-8-methoxy-3H-2,3-benzodiazepine
according to Claim 1.

3. Pharmaceutical agent containing a compound of formula I according to Claim 1 and pharmaceutically customary vehicles and excipients.

4. Process for the preparation of the compounds of formula I, according to Claim 1, **characterized in that**
a) a compound of formula II 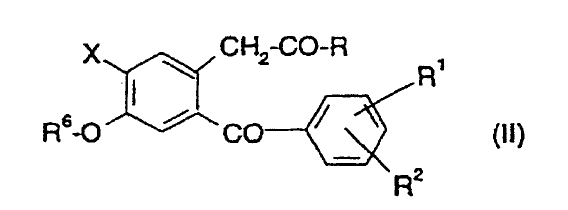 in which R¹, R², R⁶ and X have the above meaning and R is hydroxyl or C₁₋₆alkyl, is cyclized with H₂N-NH-R³ or
b) a compound of formula III 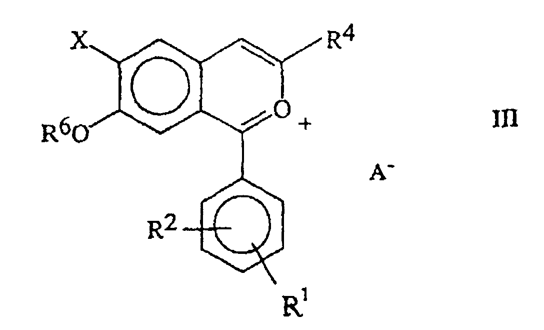 in which R¹, R², R⁴, R⁶ and X have the above meaning and A- means an anion of an inorganic base, is reacted with H₂N-NHR³ and optionally then a nitro group and/or the 3,4-double bond is reduced and/or a compound of general formula I is converted, by reduction, dehalogenation, acylation, alkylation, hydroxylation, halogenation, introduction of a carbamoyl group or an ester group, into another compound of general formula I, the isomers are separated or the salts are formed.

5. Use of the compounds according to Claim 1 or 2 for the production of a pharmaceutical agent for the treatment of diseases that are triggered by hyperactivity of excitatory amino acids.

6. Use according to Claim 5 for the symptomatic and preventive treatment of diseases that are triggered by overstimulation of the AMPA receptor.

## Revendications

1. Composés de formule I 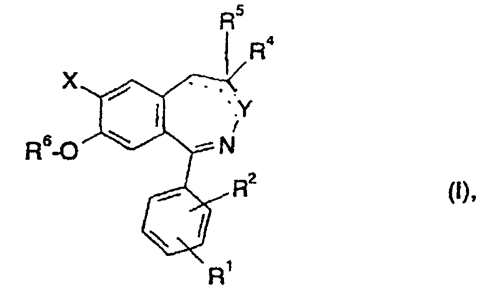 dans laquelle
X représente un atome d'hydrogène ou d'halogène,
Y représente -NR³- ou -N=,
R¹ et R² sont identiques ou différents, et représentent un atome d'hydrogène ou d'halogène ou un groupe alkyle en C₁-C₆, nitro, -NR⁸R⁹, -O-alkyle(C₁₋₄), CF₃, OH ou alcanoyloxy en C₁₋₆,
R³ représente un atome d'hydrogène, un groupe -COR¹⁰, alkyle en C₁₋₆ ou cycloalkyle en C₃₋₇,
R⁴ représente un groupe alkyle en C₁₋₆ éventuellement substitué par un atome d'halogène ou par un groupe alcoxy en C₁₋₆,
R⁵ représente un atome d'hydrogène ou
R⁴ et R⁵ représentent ensemble un atome d'oxygène,
R⁶ représente un groupe alkyle en C₁₋₄,
R⁸ et R⁹ sont identiques ou différents et représentent un atome d'hydrogène ou un groupe alkyle en C₁-C₆ ou -CO-alkyle(C₁₋₆),
R₁₀ représente un atome d'hydrogène, un groupe alkyle en C₁₋₆ éventuellement substitué par un halogène ou par un groupe alcoxy en C₁₋₆, un groupe aryle en C₆₋₁₀ éventuellement substitué par un halogène ou par un groupe alcoxy en C₁₋₆, un groupe -NR¹¹R¹², -O-alkyle(C₁₋₆), cycloalkyle en C₃₋₇, alcényle en C₂₋₆ ou -O-cycloalkyle(C₃₋₇),
R¹¹ et R¹² sont identiques ou différents et représentent un atome d'hydrogène, un groupe alkyle en C₁₋₆ éventuellement substitué par un halogène ou par un groupe alcoxy en C₁₋₆, un groupe aryle en C₆₋₁₀ éventuellement substitué par un halogène ou par un groupe alcoxy en C₁₋₆,
-C---C--- représente une double liaison ou des liaisons simples,
ainsi que leurs isomères et sels physiologiquement acceptables, à l'exclusion de la 1-(3-chlorophényl)-4-méthyl-8-méthoxy-5H-2,3-benzodiazépine.

2. 1-(4-aminophényl)-8-méthoxy-4,5-dihydro-3H-2,3-benzodiazépin-4-one,
1-(4-aminophényl)-4-méthyl-8-méthoxy-5H-2,3-benzodiazépine,
3-acétyl-1-(4-aminophényl)-4-méthyl-8-méthoxy-4,5-dihydro-3H-2,3-benzodiazépine,
3-n-propionyl-1-(4-aminophényl)-4-méthyl-8-méthoxy-4,5-dihydro-3H-2,3-benzodiazépine,
3-cyclopropylcarbonyl-1-(4-aminophényl)-4-méthyl-8-méthoxy-4,5-dihydro-3H-2,3-benzodiazépine,
3-méthoxycarbonyl-1-(4-aminophényl)-4-méthyl-8-méthoxy-4,5-dihydro-3H-2,3-benzodiazépine,
3-méthylcarbamoyl-1-(4-aminophényl)-4-méthyl-8-méthoxy-4,5-dihydro-3H-2,3-benzodiazépine,
3-acétyl-1-(4-aminophényl)-4-méthyl-8-méthoxy-3H-2,3-benzodiazépine,
3-n-propionyl-1-(4-aminophényl)-4-méthyl-8-méthoxy-3H-2,3-benzodiazépine,
3-cyclopropylcarbonyl-1-(4-aminophényl)-4-méthyl-8-méthoxy-3H-2,3-benzodiazépine,
selon la revendication 1.

3. Médicament contenant un composé de formule I selon la revendication 1 et des véhicules et adjuvants pharmaceutiquement usuels.

4. Procédé pour la préparation des composés de formule I selon la revendication 1, **caractérisé en ce que**
a) on cyclise avec H₂N-NH-R³ un composé de formule II 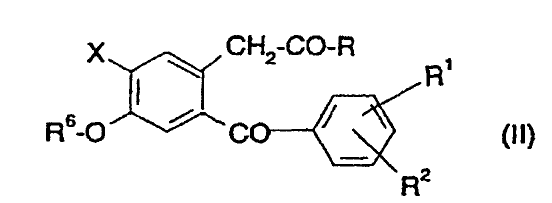 dans laquelle R¹, R², R⁶ et X ont les significations données plus haut et R est un groupe hydroxy ou alkyle en C₁₋₆ ou
b) on fait réagir avec H₂N-NHR³ un composé de formule III 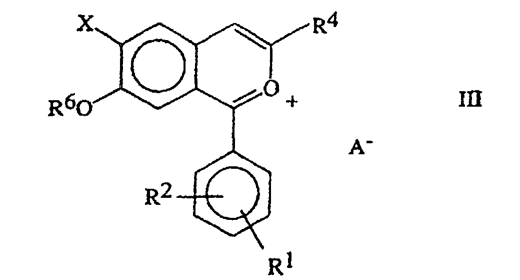
dans laquelle R¹, R², R⁴, R⁶ et X ont les significations données plus haut et A⁻ représente un anion d'une base minérale, et, si on le désire, on réduit ensuite un groupe nitro et/ou la double liaison 3,4 et/ou on convertit un composé de formule générale I en un autre composé de formule générale I par réduction, déshalogénation, acylation, alkylation, hydroxylation, halogénation, introduction d'un groupe carbamoyle ou d'un groupe ester, on sépare les isomères ou on forme les sels.

5. Utilisation des composés selon la revendication 1 ou 2, pour la fabrication d'un médicament destiné au traitement de maladies qui sont déclenchées par l'hyperactivité d'aminoacides excitateurs.

6. Utilisation selon la revendication 5, pour le traitement symptomatique et préventif de maladies qui sont déclenchées par hyperstimulation du récepteur d'AMPA.
